# EUROPEAN PATENT SPECIFICATION

(11) **EP 2 068 908 B1**
(45) Date of publication and mention of the grant of the patent: **16.07.2014**
(21) Application number: 07827108.7
(22) Date of filing: 05.10.2007
(51) Int. Cl.: A61K 35/20, A61K 38/17, A61P 29/00, A23L 1/305, A23L 1/29

(54) **A MILK PROTEIN COMPOSITION AND USE THEREOF**
MILCHPROTEIN-ZUSAMMENSETZUNG UND IHRE VERWENDUNG
COMPOSITION DE PROTÉINES DU LAIT ET UTILISATION DE CELLE-CI

(30) Priority: 06.10.2006 IE 20060734; 20.10.2006 GB 0620786
(43) Date of publication of application: 17.06.2009
(73) Proprietor: Kerry Group Services International Limited, Tralee Co. Kerry (IE)
(72) Inventor: CALAME, Wilhelmus, Hans, Albert, NL-2241 NR Wassenaar (NL); SIEMENSMA, Andries, Dirk, NL-8926 Le Leeuwarden (NL); REILLY, John, Tralee County Kerry (IE); GREANEY, Margaret, Theresa, Castleisland County Kerry (IE); FLYNN, Callaghan, Moyvane County Kerry (IE)
(74) Representative: Litton, Rory Francis
(86) International application number: PCT/IE2007/000095
(87) International publication number: WO 2008/041219

(56) References cited:
- WO-A-2006/052134

## Description

### Introduction

The invention relates to a milk protein composition for use in treating or preventing chronic local inflammation in humans. The invention also relates to the use of the milk protein composition for the manufacture of a nutritional composition and/or a pharmaceutical composition for use in treating or preventing chronic local inflammation in humans.

WO 2006/052134 discloses compositions for mitigating and preventing acute inflammatory responses and exacerbations of existing chronic inflammation.

Inflammation is a response by the body's natural defence system against foreign and/or self components and is therefore a direct action of the immune system of a host. When cells or proteins of that immune system recognise unwanted substances they become activated, and subsequent inflammation will occur.

Chronic inflammation is a condition in which the immune system is constantly triggered by something in the body of the host. It is the main contributory cause of all chronic degenerative diseases including obesity, cancer and heart disease and also plays a prominent role in ageing and autoimmune diseases. Chronic inflammation starts as an acute inflammation, which is not properly dealt with by the host immune system. Usually the host will detect this after a certain amount of time, at which time complications as a direct result of the inflammation have already started.

White blood cells, acute phase proteins and proinflammatory cytokines play a major role in the onset and progression of inflammation. With respect to the white blood cells the monocytes in the circulation which become macrophages (immune competent cells) in the tissue after leaving the blood stream, are the key cells in this process. Upon activation the macrophages will secrete a lot of proinflammatory cytokines and chemoattractants resulting in a full-blown inflammation. Macrophages, upon stimulation, are known for their capacity to release pro-inflammatory cytokines which eventually leads to the activation of the cellular and humoral (protein) components of the immune system. When such an activator is constantly present, chronic inflammation is apparent, as in the case of auto-immune diseases (e.g. rheumatoid arthritis, allergy) and in obesity.

Local inflammation is a reaction by the immune system of the host on a particular stimulus at a particular site of the body (localised) without involvement of a systemic component within the host defence. Local inflammation associated with adipose tissue is caused by a direct interaction between fat cells and macrophages. Two stages are recognised in this, first an acute phase in which the stimulus is starting to have its impact on the local host immune system and secondly a chronic phase in which the local immune system is constantly triggered due to ongoing presence of the stimulus.

Recently a connection has been established between obesity and local inflammation. In adipocytes (fat cells), the volume of the adipocytes increases as more fat, especially in triglyceride form is stored, resulting in the adipocytes secreting all kinds of stimulatory factors, such as proinflammatory cytokines including Interleukin-6 (IL-6) and Tumour Necrosis Factor-α (TNF-α), and chemoattractants, like Monocyte Chemoattractant Peptide-1 (MCP-1). These stimulatory factors direct blood monocytes to the fat tissue where they enhance the local inflammation by secreting similar products but to a higher extent than by the fat cells and by doing so they augment the local inflammatory reaction. The amount of TNF- α and IL-6 contributes to all kinds of morbidities, eventually leading to metabolic syndrome and type-2 diabetes as development progresses. TNF-α and IL-6 have been found to cause insulin resistance in animal models, and plasma levels of TNF- α and IL-6 have been shown to predict type 2 diabetes in humans.

TNF-α and IL-6 are important in inducing hepatic production of acute-phase proteins, including C-Reactive Protein (CRP). These inflammatory markers are associated with risk factors for cardiovascular events, including components of the metabolic syndrome (obesity, insulin resistance, diabetes, hypertension, and low HDL cholesterol levels), and lifestyle factors, such as smoking, abstinence from alcohol, and physical inactivity.

In general the pro-inflammatory cytokines which are being released by cells in the body will exert their full biological activity upon binding to the relevant receptors on the cell membrane of relevant cells. These receptors can be shed into the circulation where they might bind with their respective molecules inhibiting their biological action. The effects of cytokines like TNF-α are dependent on the relative abundance of the soluble receptor e.g. TNF-α with two types of molecular forms, type 1 and type 2 with the relevant receptor (sTNF-R1 and sTNF-R2), which circulate in soluble forms. The soluble receptors may attenuate the bioactivity of TNF-α but may also serve as slow-release reservoirs and promote inflammation in the absence of free TNF receptor. Other inflammatory markers, including the cytokines IL-6, and the soluble IL-6 receptor, TNF-α, TNF-α receptors, IL-8, IL-18 and IL-1 receptor antagonist, are elevated in obese people.

Obesity promotes a state of chronic low-grade inflammation, which appears to be triggered predominantly in adipose tissue which is the connective tissue specialised for fat storage. This inflammatory response is characterized by increased cytokine and acute-phase protein release, and activation of inflammatory signalling pathways. Ageing also results in an increase of inflammatory cytokines that contribute to the progression of many degenerative diseases.

A well known method of treatment or prevention of chronic inflammation is a drug-based approach. A lot of pharmaceutical drugs have been developed to interrupt the inflammatory cascade. Over-the-counter non-steroid anti-inflammatory drugs (NSAIDs) disrupt the production of prostaglandins. Corticosteroids, antihistamines and angiotensin - converting enzyme (ACE) inhibitors have followed suit, each targeted at shutting down a different inflammatory mechanism. A drawback of this drug-based approach is that the cause of the disease is not treated. Specifically, for example in the case of obesity or overweight individuals, the adipose tissue still triggers the production of the pro-inflammatory cytokines which attack and kill cells with oxidative chemicals thus promoting inflammation. Thus as soon as the individual stops taking the drugs, inflammation will reoccur. Therefore, up until now, an effective method of treatment or prevention of chronic inflammation has not been found.

The major problem with local inflammation is that it is often unnoticed and thus hard to recognize as such. Moreover, specific treatment is difficult and results in an overall treatment in which non-affected sites are also exposed. For example, by treating inflammation somewhere in the body, the beneficial aim should be achieved at a particular location in the body, i.e. for example to reduce TNF-α levels in adipose tissue, however a similar reduction in the blood stream might also occur thus causing a reduction in the immunological defence against infections. Thus at present, the attempted treatment and prevention of local inflammation results in the use of unwanted medication in general. Additionally, the recognition of local inflammation requires the application of specific tools, such as radiotracers, which have unwanted side-effects.

The treatment and/or prevention of chronic local inflammation is critical since this will develop into full blown morbidity if untreated. Thus there is a need for an improved composition for use in treating or preventing chronic local inflammation in humans.

### Statements of Invention

According to the invention, there is provided a milk protein composition for use in treating or preventing chronic local inflammation in human characterised in that the composition comprises at least 12.5% of a slow digesting milk protein by weight of the composition, said slow digesting milk protein comprising at least 50% casein by weight of the protein in micellar form.

Ideally, composition comprises between 12.5% and 95% of the slow digesting milk protein by weight of the composition. Surprisingly it has been found that slow digesting milk protein, such as casein and in particular micellar casein and caseinates, has a significant effect on inflammation in humans and in particular chronic local inflammation. By providing a composition with slow digesting milk protein, the fat cells present at the site of the localised inflammation have been found to reduce in volume and in some cases the inflammation at this localised area has been obviated.

The major difference between a fast digesting protein and a slow digesting protein is the time-dependent entry of amino acids in the circulation. Slow digesting protein consumption gives rise to a controlled, longer-lasting blood concentration of amino acids without the clear peak seen as after consumption of fast digesting proteins. Thereby the insulin release will be less pronounced in case of the former than that of the latter. This implies that the release of glucagon to ensure proper glucose levels in the circulation will be enhanced. Moreover the presence of amino acids over time by itself gives rise to glucagon production and release. Glucagon is one of the key factors in ensuring proper blood glucose levels by breaking down triglycerides in fat cells in free fatty acids. Subsequently, these free fatty acids will circulate in the body and be used as fuel in cells. Thereby fatty acid oxidation in the body will occur resulting in smaller fat cells after which the inflammatory stimulus will be minimized. This will lead to a lower production of proinflammatory cytokines and thereby a reduction in the local inflammation.

Preferably, the composition comprises at least 25% of a milk protein by weight of the composition; and wherein
the milk protein comprises at least 50% of the slow digesting milk protein by weight of the milk protein.

Further preferably, the composition comprises between 25% and 95% of a milk protein by weight of the composition; and wherein
the milk protein comprises between 50% and 99% of the slow digesting milk protein by weight of the milk protein.

Still further preferably, the milk protein comprises between 70% and 90% of the slow digesting milk protein by weight of the milk protein.

Ideally, the slow digesting milk protein comprises casein.

In one embodiment of the invention, the casein is in a micellar form and comprises one ore more of micellar casein, calcium caseinate and magnesium caseinate.

In another embodiment of the invention, the casein comprises one or more of sodium caseinate and potassium caseinate.

Preferably, the casein comprises between 40% and 100% α - casein, between 0% and 50% β- casein and between 0% and 20% κ-casein by weight of the casein.

Further preferably, the casein comprises between 53.5% and 79.2% α - casein, between 32.1% and 45.8% β- casein and between 10.7% and 16.7% κ-casein by weight of the casein.

The advantage of the milk protein composition comprising a higher percentage of α-casein is that α-caseins have been found to have a higher impact as a slow digesting protein than other types of caseins such as β-caseins. β-caseins for example stay more or less in solution at low pHs such as in the stomach, where the pH is in the region of 2.5.

K-casein is also a slow digesting protein as it forms a curd in the stomach and thus takes a longer period of time to digest. Part of the κ-casein is split by the enzyme rennin in the stomach and thus the remaining part of the κ-casein will form a curd with any fat present which takes longer to digest.

In another embodiment of the invention the composition further comprises whey protein in the amount of between 1% and 50% by weight of the milk protein.

Preferably, the composition further comprises between 0% and 5% fat, between 3% and 10% ash, between 0% and 30% carbohydrate, and between 0% and 10% moisture by weight of the composition.

Further preferably, the composition comprises between 0% and 30% lactose by weight of the carbohydrate.

Ideally, the local inflammation is low-grade local inflammation in and directed by adipose tissue. The milk protein composition has been found to be particularly effective for this type of inflammation as this inflammation is directly associated to fat cells.

According to the invention, there is also provided the use of the milk protein composition of the invention for the manufacture of a nutritional composition or a pharmaceutical composition.

According to the invention, there is further provided a nutritional composition for use in treating or preventing chronic local inflammation in humans, comprising the milk protein composition of the invention.

Preferably, the nutritional composition is selected from the group comprising a beverage product, a dessert product, a confectionary product, a baked product, a dairy product.

According to the invention, there is also provided a pharmaceutical composition for use in treating or preventing chronic local inflammation in humans, comprising the milk protein composition of the invention.

The invention further provides the use of a milk protein composition in the manufacture of a nutritional composition and/or a pharmaceutical composition for use in lowering inflammation marker levels in the blood of a human wherein the inflammation markers are selected from the group comprising one or more of Interleukin-6 (IL-6) and the soluble IL-6 receptor, Tumour Necrosis Factor- α (TNF-α), C- reactive protein (CRP), and Monocyte Chemoattractant Peptide-1 (MCP-1).

The invention still further provides the use of a milk protein composition in the manufacture of a nutritional composition and/or a pharmaceutical composition for use in lowering the Low Density Lipoprotein: High Density Lipoprotein (LDL:HDL) ratio in the blood of a human.

The invention also provides the use of a milk protein composition in the manufacture of a nutritional composition and/or a pharmaceutical composition for use in lowering the body weight and/or body fat of a human.

According to the invention there is further provided the use of a nutritional composition comprising the milk protein composition for use in lowering inflammation marker levels in the blood of a human wherein the inflammation markers are selected from the group comprising one or more of Interleukin-6 (IL-6) and the soluble IL-6 receptor, Tumour Necrosis Factor- α (TNF-α), C- reactive protein (CRP), and Monocyte Chemoattractant Peptide-1 (MCP-1).

According to the invention there is also provided the use of a nutritional composition comprising the milk protein composition for use in lowering the Low Density Lipoprotein: High Density Lipoprotein (LDL:HDL) ratio in the blood of a human.

According to the invention there is also provided the use of a nutritional composition comprising the milk protein composition for use in lowering the body weight and/or the body fat of a human.

According to the invention there is further provided the use of a pharmaceutical composition comprising the milk protein composition for use in lowering inflammation markers levels in the blood of a human wherein the inflammation markers are selected from the group comprising one or more of Interleukin-6 (IL-6) and the soluble IL-6 receptor, Tumour Necrosis Factor- α (TNF-α), C- reactive protein (CRP), and Monocyte Chemoattractant Peptide-1 (MCP-1).

According to the invention there is also provided the use of a pharmaceutical composition comprising the milk protein composition for use in lowering the Low Density Lipoprotein: High Density Lipoprotein (LDL:HDL) ratio in the blood of a human.

According to the invention there is also provided the use of a pharmaceutical composition comprising the milk protein composition for use in lowering the body weight and/or the body fat of a human.

According to the invention there is further provided a method for treating or preventing chronic local inflammation in humans which comprises administering an effective amount of the milk protein composition of the invention.

The invention further provides a method for lowering inflammation marker levels in the blood of a human, wherein the inflammation markers comprises one or more of Interleukin-6 (IL-6) and the soluble IL-6 receptor, Tumour Necrosis Factor-α (TNF-α), C- reactive protein (CRP), and Monocyte Chemoattractant Peptide - 1 (MCP-1) and wherein the method comprises administering an effective amount of the milk protein composition of the invention.

The invention still further provides a method for lowering the LDL:HDL ratio in the blood of a human which comprises administering an effective amount of the milk protein composition of the invention.

The invention also provides a method for lowering the body weight and/or body fat of a human which comprises administering an effective amount of the milk protein composition of the invention.

### Detailed Description of the Invention

The invention will now be more clearly understood from the following drawings in which:
Figure 1 depicts a change in bodyweight during 12 weeks of intervention as described in Example 1;
Figure 2 depicts a change in body fat during 12 weeks of intervention as described in Example 2;
Figure 3 depicts a change in LDL:HDL ratio in blood serum during 12 weeks of intervention as described in Example 3;
Figure 4 depicts a change in C-reactive Protein in blood serum during 12 weeks of intervention as described in Example 4;
Figure 5a depicts a change in IL-6 in blood serum during 12 weeks of intervention as described in Example 5;
Figure 5b depicts a change in soluble IL-6 receptor IL-6 analogue in blood serum during 12 weeks of intervention as described in Example 5;
Figure 6 depicts a change in TNF - α in blood serum during 12 weeks of intervention as described in Example 6.
Figure 7 depicts a change in MCP-1 in blood serum during 12 weeks of intervention as described in Example 7.

The invention will be also be more clearly understood from the following description of one method of manufacturing the milk protein composition according to the invention.

In order to obtain the milk protein composition, skim milk is subjected to lactose separation by either filtration or precipitation technology to provide milk protein and lactose. The milk protein is then subjected to heat treatment and drying to provide dried milk protein. Heat treatment should be carried out at suitable temperatures to avoid protein denaturation. Additional ingredients as outlined below can then be added to the milk protein to provide the milk protein composition. In the specification the term "milk protein composition" refers to a mixture of components, wherein at least 25% of the composition comprises milk protein. It will be appreciated that whole milk could also be used to prepare the milk protein composition, however whole milk would need to be firstly subjected to a fat separation process for cream removal.

In the specification the term "slow digesting milk protein" refers to milk protein with a slow digestion rate. This type of protein is held in the stomach after ingestion and is transported at a later stage to the small intestine where absorption of the peptides and free amino acids takes place. As a result the amino acids appear at a later time in the blood which results in a lower state of local inflammation by a mechanism described in more detail later. The slow digesting protein should comprise at least 50% casein by weight of the protein and should be in a micellar form such as micellar casein, calcium caseinate, or magnesium caseinate, or in the form of sodium caseinate or potassium caseinate.

In the specification the term "micellar casein" refers to casein in the form of micelles. Casein micelles are large macromolecular aggregates and contain a large proportion of protein, generally between 90% and 95% by weight, with the remainder comprising inorganic salts, in particular calcium phosphate. The micelles generally have a hydrodynamic radius of 40-400nm, a molecular weight of 10⁶ - 10⁹ Daltons and a calcium to phosphorus weight ratio of 1.4 - 2.4. Casein micelles generally comprise four types of casein, namely αₛ₁-, αₛ₂-, β- and κ-caseins. The normal ratio in cows milk of α-casein -v- β-casein is 4:3, however variations of from 5:3 to 2:1 are possible. The micellar casein clots under acidic gastric pHs which delays the amino acid delivery from the stomach to the intestine and as a result the amino acids appear at a later time in the blood which results in a lower state of local inflammation as described later.

In the specification, the term "caseinate" refers to casein which has been bound to a metal, the result being a more stable molecule. Specifically, casein is most commonly bound to calcium (Ca²⁺) or sodium (Na⁺) since both of these metals are found naturally in milk, and tend to "stick" to the casein during the extraction process. Nutritionally, these compounds are interchangeable, as both forms of caseinate are effective protein sources. Casein can also bind to potassium (K⁺) and magnesium (Mg²⁺) to form potassium caseinate and magnesium caseinate respectively. The caseinates precipitate under acidic gastric pHs which delays the amino acid delivery from the stomach to the intestine and as a result the amino acids appear at a later time in the blood which results in a lower state of local inflammation as described later. Calcium caseinate and magnesium caseinate are also forms of micellar casein.

In the case of micellar casein, it has been found that larger micelles i.e. micelles greater than 200nm in diameter are more preferable as they take a longer time to digest and thus are slower digesting proteins than smaller micelles.

It has been found that the mechanism by which these slow digesting milk proteins work to reduce local inflammation is as follows: The consumption of slow digesting milk proteins leads to a time-dependent entry of amino acids from the proteins into the blood circulation. The presence of the amino acids, leads to insulin and glucagon also being released into the blood circulation, which results in lower glucose levels in the circulation due to the cellular uptake by the organs. The result is a relative shortage of glucose to fulfil the energetic needs in the body. In order to overcome this shortage of glucose, triglycerides in fat deposits (adipose tissue) are being used for energy purposes under the influence of glucagon. This leads to the release of free fatty acids into the circulation which are used for energy by the rest of the body, and the associated shrinking of enlarged fat cells in the adipose tissue. These local fat cells have reduced activation and the release of pro-inflammatory cytokines are thereby minimised resulting in a lower state of local inflammation.

As well as comprising a high proportion of casein the composition may also comprise whey, soy proteins, egg proteins, other milk proteins and combinations of these.

The carbohydrate source in the milk protein composition generally comprises a percentage of lactose as it has a low glycemic response. Foods with low glycemic responses lead to a smaller rise in blood glucose and insulin. This promotes satiety and results in a lower weight gain than carbohydrates in a food having a high glycemic response because they take longer to be absorbed. Other carbohydrates such as starch, maltodextrin, isomaltose, glucose syrup, fructose or any other suitable carbohydrate may be included.

The composition can also include a source of fibre which may be in the form of barley flakes, oat fibre, pea fibre, soy fibre, konjac mannan, psyllium, inulins, guar gum, locuse bean gum, carrageenan, arabic gum, alginate, resistance starches, soy fibre, pectin, fructo-oligosaccharides, gluco-oligosaccharides, modified cellulose, polyalcohols such as xylitol and combinations thereof.

The fat source can include vegetable oils such as canola oil, olive oil, soybean oil, sunflower oil, safflower oil, corn oil sesame seed oil, evening primrose oil, peanut oil, cottonseed oil, high oleic sunflower oil, rapeseed oil, coconut oil, macadamia oil, palm oil, palm kernel oil, or fish oils such as cod liver oil, sardine oil, menhaden oil, medium chain triglycerides (MCT), and esters of fatty acids wherein the fatty acids are, for example, arachidonic acid, linoleic acid, palmitic acid, stearic acid, docosahexaeonic acid, eicosapentaenoic acid, linolenic acid, oleic acid, lauric acid, capric acid, caprylic acid, caproic acid and combinations thereof. The fat source can also be any type of animal fat such as butter oil, milk fat, and egg yolk lipid.

Ash generally comprises milk minerals such as sodium, potassium, calcium, chloride, magnesium and phosphorus.

The milk protein composition is provided in the form of the powder. The powder can either be instantised so that can be readily dissolved in beverages. Alternatively, the powder can be agglomerated to aid dispersion and solubility of the powder.

It is proposed to sell this milk protein composition under the Trade Mark Ultranor Bio-M^{™}. Ultranor Bio-M^{™} comprises a number of compositions all of which comprise in the region of between 25% and 95% casein by weight of the compositions.

The following examples of typical milk protein compositions which will be sold under the Trade Mark Ultranor Bio-M^{™} are given by way of illustration only and should not be construed as limiting the subject matter of the invention. The protein component in each of the products has a casein : whey ratio of approximately 85 : 15.

**Table 1: Ultranor Bio-M^{™} Milk protein Composition (∼59.5% Micellar casein)**

| **Nutrient** | **Amount (%)** |
|---|---|
| Protein | 70.0 |
| Fat | 1.3 |
| Moisture | 5.7 |
| Ash | 7.0 |
| Carbohydrate (lactose) | 16.0 |

**Table 2: Ultranor Bio-M^{™} Milk protein Composition (∼61.2% Magnesium caseinate)**

| **Nutrient** | **Amount (%)** |
|---|---|
| Protein | 72.0 |
| Fat | 5.0 |
| Moisture | 9.0 |
| Ash | 7.5 |
| Carbohydrate (lactose) | 6.5 |

**Table 3: Ultranor Bio-M^{™} Milk protein Composition (∼71.4% Sodium caseinate)**

| **Nutrient** | **Amount (%)** |
|---|---|
| Protein | 84.0 |
| Fat | 1.1 |
| Moisture | 4.0 |
| Ash | 7.0 |
| Carbohydrate (lactose) | 3.9 |

**Table 4:Ultranor Bio-M^{™} Milk protein Composition (∼72.25% Potassium caseinate)**

| **Nutrient** | **Amount (%)** |
|---|---|
| Protein | 85.0 |
| Fat | 3.2 |
| Moisture | 4.8 |
| Ash | 6.0 |
| Carbohydrate (lactose) | 1.0 |

**Table 5: Ultranor Bio-M^{™} Milk protein Composition (∼73.95% Micellar casein)**

| **Nutrient** | **Amount (%)** |
|---|---|
| Protein | 87.0 |
| Fat | 0.0 |
| Moisture | 10.0 |
| Ash | 3.0 |
| Carbohydrate | 0.0 |

**Table 6: Ultranor Bio-M^{™} Milk protein Composition (~74.8% Calcium caseinate)**

| **Nutrient** | **Amount (%)** |
|---|---|
| Protein | 88.0 |
| Fat | 1.5 |
| Moisture | 4.0 |
| Ash | 6.0 |
| Carbohydrate (lactose) | 0.5 |

It will be appreciated, however, that other milk protein compositions having a casein content within this range could be used in order to achieve the desired effect of the invention.

The milk protein composition can be used in the manufacture of many types of nutritional compositions such as food and beverage products. Suitable types of food or beverage products could include, liquid concentrates, fruit juice beverages, ready-to-drink beverages, vegetable juice products, isotonic drinks, carbonated flavoured drinks, milk or milk products, ice-cream or frozen based confections, dessert products, fermented milk products such as yoghurt or quark, cheeses, soups, sauces, ready meals, confections such as chocolate, biscuits, bars or any other food or beverage product which delivers protein in a form fit for consumption. The following examples of products are given by way of illustration only and should not be construed as limiting the subject matter of the invention.

The following products are manufactured according to the specifications of the manufacturer, with the exception that all or some of the standard protein generally used to prepare these products, e.g. whey protein isolate or soya isolate, is replaced by the milk protein composition of the invention.

**Table 7: Dry Mix High Protein Flavoured Milk Shakes**

| **Ingredients** | **Dry Mix %** | **Final Drink %** |
|---|---|---|
| Ultranor Bio-M™ | 96.87 | 29.06 |
| Flavour | 0.94 | 0.28 |
| Sherex® CM9585 Texturiser (stabiliser) | 0.80 | 0.24 |
| Colour (Carmine WS5) | 0.54 | 0.16 |
| Sucralose | 0.077 | 0.02 |
| Salt | 0.80 | 0.24 |
| Water | | 70.00 |

**Table 8: Sugar Free Dairy Ice-Cream**

| **Ingredient** | **%** |
|---|---|
| Cream - 40% Fat | 20.0 |
| Ultranor Bio-M™ | 15.00 |
| Maltitol Syrup (63% solids) | 10.0 |
| Sorbitol | 5.0 |
| Sherex® I 9535 | 0.6 |
| Vanilla Flavour | 0.15 |
| Colour -B-carotene | 0.005 |
| Acesulfame K | 0.012 |
| Aspartame | 0.012 |
| Water | to 100 |

**Table 9: High Protein Nutrition Bars**

| **Ingredient** | **%** |
|---|---|
| Maltitol syrup (75%) | 24.73 |
| Ultranor Bio-M™ | 28.41 |
| Polydextrose | 9.82 |
| Vegetable Fat Fractionated Palm Kernal oil (PKO) | 4.46 |
| Glycerine | 2.63 |
| Soy Protein Crisps (Crushed) | 9.16 |
| Coconut fruit | 4.72 |
| Admul® PGE Emulsifier | 0.20 |
| Sucralose (20% soln) | 0.037 |
| Salt | 0.133 |
| Marshmallow flavours | 0.70 |
| Chocolate coating | 15.0 |

**Table 10: High Protein Shake**

| **Ingredient** | **%** |
|---|---|
| Ultranor Bio-M™ | 11.4 |
| Beatreme™ Beverage Base | 5.0 |
| Cocoa | 2.25 |
| Chocolate Powder | 2.25 |
| Sucralose | 0.008 |
| Mastertaste Chocolate Flavours | to taste |
| Mastertaste Strawberry Flavours | to taste |
| Water | to 100 |

**Table 11: Reduced Calorie Drinking Yoghurt**

| | **Yoghurt Base** | **Drinking Yoghurt** |
|---|---|---|
| **Ingredient** | **%** | **%** |
| Ultranor Bio-M™ | 31.0 | 23.25 |
| Cream (40% fat) | 5.0 | 3.8 |
| Probiotic Cultures | 0.002 | 0.0015 |
| Water | 63.998 | 47.95 |
| Fruit Preparation | | 22.0 |
| PreVitae™ Fibre | | 3.0 |

Each of the food products has a recommended serving size so as to provide in the region of 25g of protein per serving of which between 50% and 99% and preferably between 70% and 90% is casein. Thus on average, 20g of casein is provided by the advised amount of each food product. It is also recommended that two of these food products are consumed daily so as to achieve the desired effect.

The above amounts have been found to be most effective for those individuals weighing between 85 and 110kg. It will be appreciated however that for individuals weighing more or less than this range that these amounts will also be effective.

The pharmaceutical composition can be provided in many forms such as tablet, powder, suspension, liquid, capsule, or gel and as well as comprising the milk protein composition should also include a suitable carrier or excipient.

The pharmaceutical composition can also include vitamins, minerals antioxidants and other nutritional supplements, which should be included in amounts according to nutritional recommendations.

Vitamins and minerals that can be added include, but are not limited to calcium phosphate or acetate, potassium phosphate, magnesium sulphate, sodium chloride, potassium chloride, ascorbic acid, ferric orthophosphate, niacin amide, zinc sulphate, calcium pantothenate, copper glucanate, riboflavin, beta-carotene, pyridoxine hydrochloride, thiamine mononitrate, folic acid, biotin, chromium chloride, potassium iodide, selenium, sodium selenate, sodium molybdate, phylloquinone, cyanocobalamin, sodium selenite, copper sulphate, potassium iodide, Vitamin A, Vitamin B6 and hydrochloride thereof, Vitamin B12, Vitamin C Vitamin D, vitamin E, Vitamin K, and any other additives listed in the Commission Directive 2001/15/EC of February 15, 2001 which lists the substances that may be added for specific nutritional purposes in foods for particular nutritional uses.

The pharmaceutical composition can also comprise fibres and oils and fats such as Omega 3 oil, fish oil, medium chain triglyceride (MCT) oil, linseed oil and esters of fatty acids wherein the fatty acids are, for example, arachidonic acid, linoleic acid, palmitic acid, stearic acid, docosahexaeonic acid, eicosapentaenoic acid, linolenic acid, oleic acid, lauric acid, capric acid, caprylic acid, caproic acid, and the like.

The pharmaceutical composition can either be administered orally or enterally.

### Trial

During the first five weeks of the trial, all subjects were fed a very low calorie diet (VLCD). Subsequent to this each of the subjects had one week to adapt during which time they were allowed to eat or drink any type of food or beverage that they wished while also focussing on keeping their weight down.

### Intervention Period

### Subjects

Based upon power analysis, each group consisted of at least 20 subjects, therefore the final number of subjects recruited was 60.

### Inclusion criteria:

- 30-60 years
- Gender: males and females (not pregnant or lactating) Body Mass Index (BMI) ≥27 kg/m²
- Smokers and non-smokers
- No weight changes over the last 3 months
- General healthy status as determined at screening

### Exclusion criteria:

- Pregnant/lactating females/women planning a pregnancy in the next 12 months. Women who became pregnant during the dietary intervention period were excluded from the study
- Elevated fasting blood glucose levels (>7mmol/l)
- BMI≤27 kg/m²
- Weight changes >5 kg over the last 3 months
- Elevated blood pressure
- Diabetes or impaired glucose tolerance
- Prescript drug medication
- Alcohol or drug abuse (based on clinical judgment)

Before subjects were allowed to participate in the study they were screened by means of a health questionnaire conducted by a physician. Furthermore, blood pressure was measured and a fasting blood sample was taken in order to determine glucose levels. When subjects scored positive here, further examination was done to determine whether the subject was to be excluded or not.

Subjects were removed from the study if the investigator became aware of any of the following conditions:
- Pregnancy
- Non-medical reasons (e.g. subjects' request or non-compliance with the treatment regimen)
- Medical reasons considered significant by the subject and/or investigator
- Protocol violation

The nature and risks of the experimental procedures, like a small bruise at the site of venapunction, were explained to the subjects and their informed consent was obtained.

Subjects that were eligible for enrolment were assigned to one of the intervention groups in a random order. The two intervention groups comprised (a) a medium fat, high protein diet and (b) a medium fat, high carbohydrate diet. The first group, hereinafter referred to as Protein Group (PG), were fed a diet comprising 30% fat, 25% protein and 45% carbohydrate. The second group, hereinafter referred to as carbohydrate group (CG) were fed a diet comprising 30% fat, 15% protein and 55% carbohydrate. In the PG diet the 25% protein was either casein or whey protein dominated. In the casein dominated group the subjects were provided with a selection of food and beverage products comprising a milk protein composition which will be sold under the Trade Mark Ultranor Bio-M^{™}. The food products comprised in the region of between 80% and 90% casein by weight of the product. In the whey dominated protein group the subjects were given a powder containing in the region of 80% whey which had been dissolved in water. In the CG diet, a maltodextrin drink was used to enhance the carbohydrate intake to reach the 55% in carbohydrate intake.

A trained dietician firstly determined a subjects' daily energy intake, by means of a weighed daily checklist of all foods, which was used afterwards to give him/her dietary advise. Subjects are asked to fill out the same daily checklist after 1, 2, 4 and 6 weeks of intervention to determine whether subjects indeed followed the dietary advise and, if necessary, to adapt the advice.

Apart from the dietary counselling, subjects were asked to consume food products which were either protein rich food products or carbohydrate rich alternatives depending on the group that they are assigned to. It was recommended that products should be used during breakfast while for consuming the other protein rich or carbohydrate products according to their group subjects were free to consume whenever they felt like eating something.

Subjects were obligated to eat two experimental products a day. One of the experimental products either had to be eaten as breakfast or with breakfast. The other experimental product had to be eaten in the afternoon between lunch and dinner. Subjects were, during dietary counselling, advised which products commercially available could be used in either the PG and CG group in order to achieve the previously described dietary change and further they were provided with high protein or high carbohydrate food products which for example the milk protein dominated protein group were the food products comprising the Ultranor Bio M^{™} compositions such as drinks and snack bars to consume during the meals or in between the meals, if they felt hungry.

### Testing procedures

Blood samples (4ml whole blood in Ethylene Diamine Tetra Acetic acid (EDTA) collecting tubes) were taken at 0, 2, 4, 8 and 12 weeks of intervention. These blood samples were used for glucose, insulin, glucagon, free fatty acids, Low Density Lipoprotein (LDL), High Density Lipoprotein (HDL), triglycerides and Haemoglobin A1 c (Hba1c) determination. In addition, at month 0, 2, 4 and 6 extra blood (10ml whole blood in EDTA collecting tubes) were withdrawn for determination of inflammatory markers (Interleukins IL-1, IL-6, Tumour Necrosis Factor (TNF-α) and C-reactive Protein (CRP)), Interleukins IL-1b, IL-4, IL-5, IL-8, IL-10, IL-12, IL-17, IL-18, Interferon IFN-gamma, Tumour Necrosis Factor TNF-β, Monocyte chemoattractant protein MCP-1, macrophage inflammatory proteins MIP-1α and MIP-1β, Granulocyte-macrophage colony-stimulating factor GM-CSF, Neurotrophins NT-3 and NT-4, Triggering Receptor expressed on myeloid cells TREM-1, Brain-Derived Neurotrophic Factor BDNF, Transforming Growth Factor TGF-β, soluble Tumour Necrosis Factor receptors sTNF R1, Matrix Metalloproteinase 9 (MMP-9), soluble Interleukin s-IL-6ra, chemokine RANTES (regulated upon activation, normal T-cell expressed and presumably secreted), and adiponectin.

Furthermore, weight (with an accuracy of 0.01 kg), hip- and waist circumference and blood pressure were measured weekly until 12 weeks of intervention. In addition body composition was also determined by means of underwater weighing. In this way, the percentage body fat was calculated from the whole body density, which is calculated by using the subject's weight under water combined with the subject's laboratory weight (2-compartment model). Finally, subjects were asked to collect 24h urine in order to determine urinary nitrogen. In this way compliance to the diet was controlled.

### Body Composition Extension

Fifteen volunteers per group underwent underwater weighing after 0, 4 and 6 months of intervention in order to determine their body composition. In this subpopulation, body composition was estimated from a three-compartment model, which divides the body into fat mass, total body water and the remaining solids of the fat free mass (predominately protein and minerals) in the method described in Ellis, K.J., Human body composition: in vivo methods. Physiol Rev, 2000;80(2):649-80. The percentage body fat was calculated from body density, measured with underwater weighing. The subject was completely submerged in water and the subject's weight under water combined with the subject's laboratory weight were used to calculate the whole body density in the method described in Ellis, K.J., Human body composition: in vivo methods. Physiol Rev, 2000;80(2):649-80.

Total body water was determined by deuterium dilution according to the Maastricht protocol. Subjects drank a small amount of labelled water in the evening after collecting a baseline urine sample. A second sample was collected the next morning after a 10-hour equilibration time.

### Biochemical methods

### Blood samples

Plasma glucose, total free fatty acids, triacylglycerol, total cholesterol. HDL and LDL cholesterol and CRP were measured in EDTA plasma on a Cobas FARA semi-automated analyzer (Roche, Basel, Switzerland). Insulin, glucagon and CCK concentrations were measured by means of a radio immunoassay (RIA), using specific double antibody. GLP-1, IL-1, IL-6 and TNF-α were determined via Enzyme linked Immunosorbent Assay (ELISA). Glycated haemoglobin (HbA_{1c}) was measured in whole blood by making use of high-pressure liquid chromatography (HPLC) with an ion exchanger (Biorad, UK).

### Analysis of other factors

Levels of Brain-Derived Neurotrophic Factor, Neurotrophic Factor-3 and -4 were also analysed via enzyme-linked immunosorbent analysis at the same time-points as the previously discussed analyses and the concentration of the brain-derived factors were found to range between 10 and 20, 70 and 95, 9 and 12 ng/ml, respectively, during the course of the experiment. These values demonstrated that a change in the concentration of the pro-inflammatory cytokines (TNF-alpha, MCP-1 and IL-6) were not associated with a change in the concentration of the respective brain derived factors. Thus using a combination of the response between proinflammatory cytokines and brain-derived factors one can distinguish local from systemic inflammation.

### Examples

The following examples are given by way of illustration only and should not be construed as limiting the subject matter of the invention.

### Example 1: Bodyweight reduction during 12 weeks of intervention:

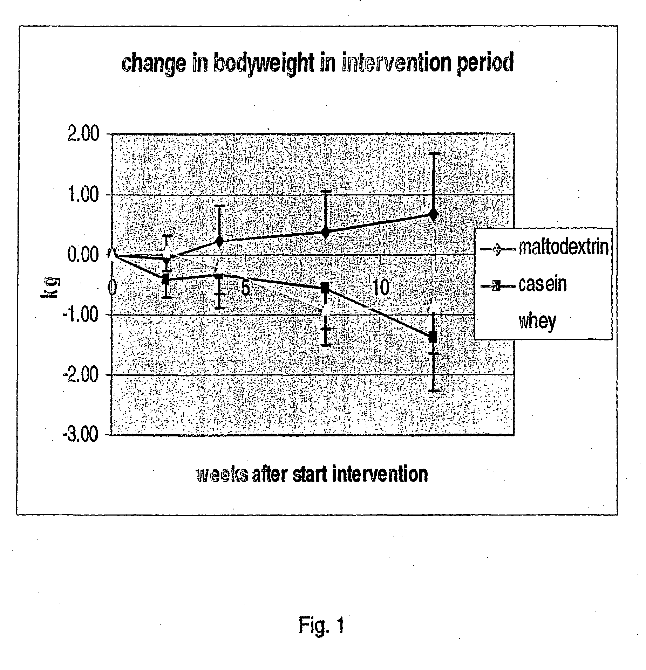

It was found that the subjects who consumed either casein or whey protein lost weight and were approximately 1.5 - 2.0 kg lighter than those who consumed maltodextrin. The more obese a subject the higher the weight loss found. The results of this example are illustrated in Fig. 1.

### Example 2: Bodyfat reduction during 12 weeks of intervention:

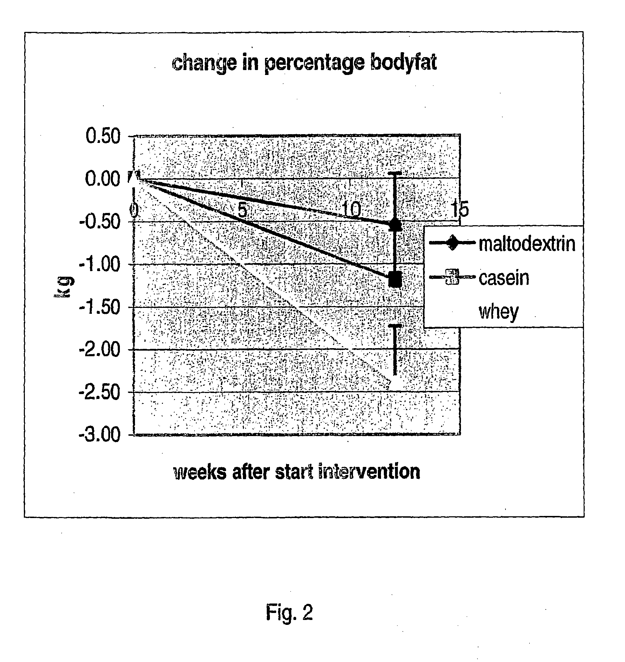

It was found that all subjects lost body fat during the trial. Again the more obese the higher the reduction in body fat. The group of subjects belonging to the whey dominated protein group were found to experience the most significant loss in body fat. This is consistent with the literature, which promotes whey proteins and peptides derived therefrom in preventing or treating obesity. The results of this example are illustrated in Fig. 2.

### Example 3: Blood cholesterol levels using LDL:HDL ratio's

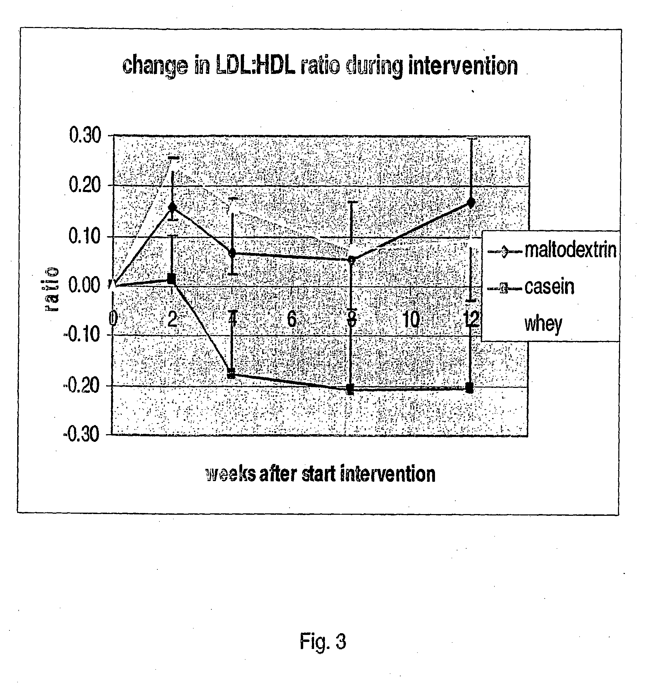

A reduction in the LDL:HDL ratio was observed for those subjects in the casein dominated protein group compared to that of whey dominated and maltodextrin groups. It is striking that the 10% reduction in body weight upfront yields a reduction in the above mentioned ratio as well, and that consumption of casein afterwards reduces this ratio even further. The major effect of casein on this ratio was due to a significant increase in HDL concentrations. The results of this example are illustrated in Fig. 3.

### Analysis of Inflammation Markers

Obesity is considered to be a disease with upraised levels of various components of the immune system. Fat cells in obese people are bigger and as a result of that secrete various factors attracting immune-competent cells, like monocytes/macrophages. Once the macrophages are stimulated by the local production of proinflammatory cytokines they start releasing the same compounds but then to a larger extent. By doing so the local inflammatory reaction is complete and might lead to all kinds of comorbidities if untreated. Since the immune competent cells are being recycled a constant flow from the circulation is occurring. By specific protein consumption the volume of the fat cells will decrease and thereby the stimulatory activity. Finally the entry (diapedesis) of immunecompetent cells will diminish and the local inflammatory condition will end.

### Example 4: Inflammation marker: C-reactive protein.

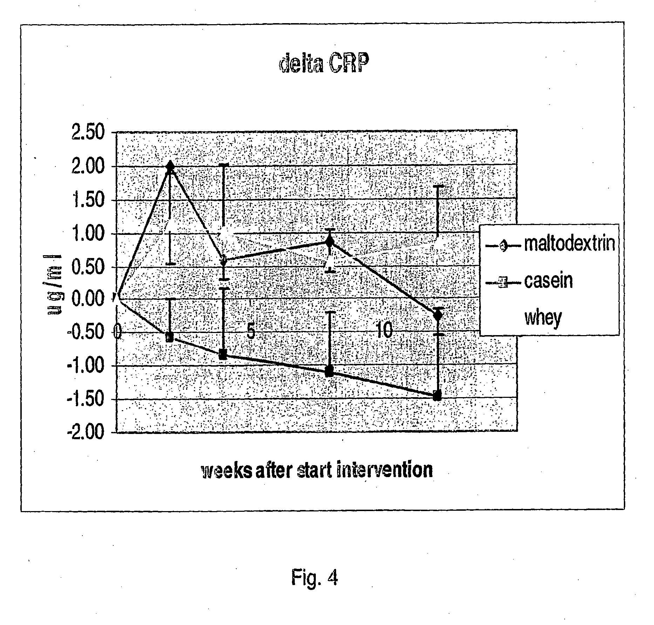

C-reactive protein is a marker of activation of the immune system. It was found that the subjects in the casein dominated protein group experienced the greatest decrease in the concentration of C-reactive protein in the circulation reflecting a less inflamed condition. This reduction is more significant than observed at the 10% weight loss initially. The results of this example are illustrated in Fig. 4.

### Example 5: Inflammation marker: Interleukin-6 (IL-6)

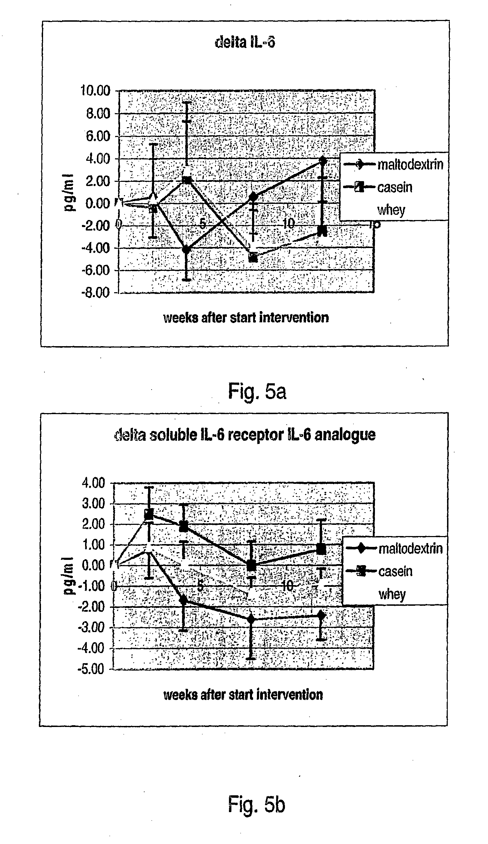

Interleukin-6 is a proinflammatory molecule, secreted by many cell types such as macrophages, endothelial cells and fat cells and indicates a state of inflammation. Soluble IL-6 can be inactivated by coupling to soluble IL-6 receptors in the circulation. Therefore, ideally, IL-6 levels should be low while those of IL-6 receptors high. The results of this example are illustrated in Figs. 5a and 5b.

It was found that the subjects in the casein dominated protein group had lower levels of IL-6 as well as significantly higher levels of soluble IL-6 receptors and thereby indicating reduced inflammation. The results for IL-6 in the whey dominated protein group were found to be similar, but the results of the IL-6 receptors were lower than that for casein thus indicating a higher inflammatory status. Maltodextrin was found to be ineffective.

### Example 6: Inflammation marker: Tumour Necrosis Factor α (TNF-α)

Tumour Necrosis Factor- α (TNF-α) is a biomarker of inflammation and occurs immediately after the onset of inflammation. This molecule is released within 30-60 minutes after the stimulus.

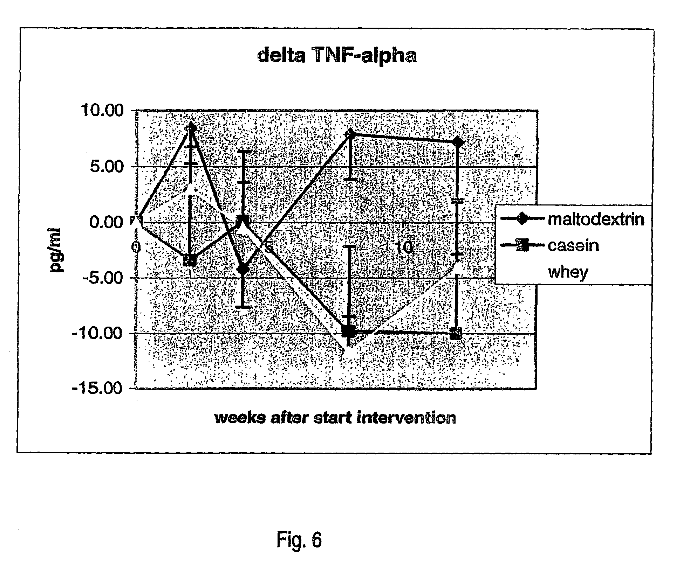

It was found that subjects in both the casein dominated and whey dominated protein groups experienced a significant drop in the concentration of TNF-α in the blood with the highest drop found in the casein consuming volunteers. The results of this example are illustrated in Fig. 6.

### Example 7: Inflammation marker: Monocyte Chemoattractant Protein 1 (MCP-1)

MCP-1 is a chemotaxin protein chemoattractants secreted by various cell types, such as macrophages and fat cells in order to attract immune-competent cells to the site of inflammation. A reduction in the concentration of MCP-1 signifies a reduction in the state of inflammation as determined via blood concentrations.

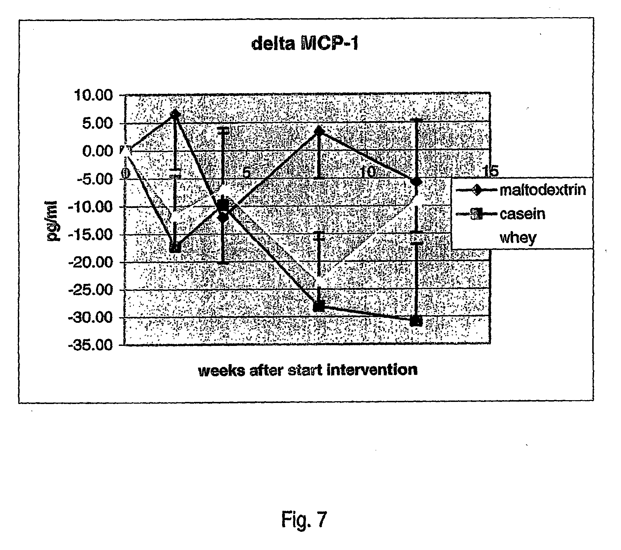

It was found that subjects in both the casein dominated and whey dominated protein groups experienced a significant drop in the concentration of MCP-1 in the blood with the highest drop found in the casein consuming category. The results of this example are illustrated in Fig. 7.

In summary, it was found that within the experimental set-up using overweight/obese persons by consuming 50 grams of proteins (casein-dominated or whey-dominated) or carbohydrates (maltodextrin) per day for 12 weeks the casein-dominated group reduced the bodyweight in contrast to the carbohydrate group and more substantially and longer than the whey-dominated group. This was accompanied by a reduction in cholesterol levels and a reduction in the local inflammatory status of the volunteers. This local inflammatory process originates from expanding fat cells. This reduction was demonstrated by the profile of various proinflammatory cytokines and chemoattractants, like, TNF-α, IL-6, soluble IL-6 receptors, MCP-1 without any effect on nerve-related factors, like BDNF, NF-3 and NF-4. These findings demonstrate that in a local inflammatory reaction can be down regulated by a diet consisting of slow digesting protein such as casein-dominated proteins.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiment hereinbefore described, but may be varied in both construction and detail within the scope of the claims.

## Claims

1. A milk protein composition for use in treating or preventing chronic local inflammation in humans **characterised in that** the composition comprises at least 12.5% of a slow digesting milk protein by weight of the composition, said slow digesting milk protein comprising at least 50% casein by weight of the protein in micellar form.

2. A milk protein composition for use as claimed in claim 1, wherein
the composition comprises at least 25%, preferably between 25% and 95% of a milk protein by weight of the composition; and wherein
the milk protein comprises at least 50%, preferably between 50% and 99%, even more preferably between 70% and 90% of the slow digesting milk protein by weight of the milk protein.

3. A milk protein composition for use as claimed in any preceding claim, wherein the slow digesting milk protein comprises casein, preferably wherein the casein is in a micellar form and comprises one or more of micellar casein, calcium caseinate and magnesium caseinate.

4. A milk protein composition for use as claimed in claim 3, wherein the casein comprises one or more of sodium caseinate and potassium caseinate.

5. A milk protein composition for use as claimed in claims 3 or 4 wherein the casein comprises between 40% and 100% α - casein, between 0% and 50% β-casein and between 0% and 20% κ-casein by weight of the casein.

6. A milk protein composition for use as claimed in any of claims 3 to 5 wherein the casein comprises between 53.5% and 79.2% α - casein, between 32.1 % and 45.8% β- casein and between 10.7% and 16.7% κ-casein by weight of the casein.

7. A milk protein composition for use as claimed in any of claims 2 to 6, wherein the composition further comprises whey protein in the amount of between 1% and 50% by weight of the milk protein.

8. A milk protein composition for use as claimed in any preceding claim, wherein the composition further comprises between 0% and 5% fat, between 3% and 10% ash, between 0% and 30% carbohydrate, and between 0% and 10% moisture by weight of the composition.

9. A milk protein composition for use as claimed in claim 8, wherein the composition comprises between 0% and 30% lactose by weight of the carbohydrate.

10. A milk protein composition for use as claimed in any preceding claim, wherein the local inflammation is low-grade local inflammation in and directed by adipose tissue.

11. Use of the milk protein composition as claimed in any of claims 1 to 10 for the manufacture of a nutritional composition or a pharmaceutical composition for treating or preventing local inflammation in humans.

12. A nutritional composition for use in treating or preventing chronic local inflammation in humans, comprising the milk protein composition as claimed in any of claims 1 to 10.

13. A nutritional composition for use in treating or preventing chronic local inflammation in humans as claimed in claim 12 wherein the nutritional composition is selected from the group comprising a beverage product, a dessert product, a confectionary product, a baked product, a dairy product.

14. A pharmaceutical composition for use in treating or preventing chronic local inflammation in humans, comprising the milk protein composition as claimed in any of claims 1 to 10.

## Patentansprüche

1. Milchproteinzusammensetzung für die Verwendung bei der Behandlung oder Prävention von chronischer lokaler Entzündung bei Menschen, **dadurch gekennzeichnet, dass** die Zusammensetzung mindestens 12,5 % eines langsam verdaulichen Milchproteins, bezogen auf das Gewicht der Zusammensetzung, umfasst, wobei das genannte langsam verdauliche Milchprotein mindestens 50 % Casein, bezogen auf das Gewicht des Proteins in mizellarer Form, umfasst.

2. Milchproteinzusammensetzung für die Verwendung nach Anspruch 1, worin
die Zusammensetzung mindestens 25 %, bevorzugt zwischen 25 % und 95 % eines Milchproteins, bezogen auf das Gewicht der Zusammensetzung, umfasst; und worin
das Milchprotein mindestens 50 %, bevorzugt zwischen 50 % und 99 %, noch bevorzugter zwischen 70 % und 90 % des langsam verdaulichen Milchproteins, bezogen auf das Gewicht des Milchproteins, umfasst.

3. Milchproteinzusammensetzung für die Verwendung nach einem vorstehenden Anspruch, worin das langsam verdauliche Milchprotein Casein umfasst, worin bevorzugt das Casein in mizellarer Form vorliegt und eines oder mehrere von mizellarem Casein, Calciumcaseinat und Magnesiumcaseinat umfasst.

4. Milchproteinzusammensetzung für die Verwendung nach Anspruch 3, worin das Casein eines oder mehrere von Natriumcaseinat und Kaliumcaseinat umfasst.

5. Milchproteinzusammensetzung für die Verwendung nach Anspruch 3 oder 4, worin das Casein zwischen 40 % und 100 % α-Casein, zwischen 0 % und 50 % β-Casein und zwischen 0 % und 20 % κ-Casein, bezogen auf das Gewicht des Caseins, umfasst.

6. Milchproteinzusammensetzung für die Verwendung nach einem der Ansprüche 3 bis 5, worin das Casein zwischen 53,5 % und 79,2 % α-Casein, zwischen 32,1 % und 45,8 % β-Casein und zwischen 10,7 % und 16,7 % κ-Casein, bezogen auf das Gewicht des Caseins, umfasst.

7. Milchproteinzusammensetzung für die Verwendung nach einem der Ansprüche 2 bis 6, worin die Zusammensetzung weiter Molkenprotein in der Menge von zwischen 1 % und 50 %, bezogen auf das Gewicht des Milchproteins, umfasst.

8. Milchproteinzusammensetzung für die Verwendung nach einem vorstehenden Anspruch, worin die Zusammensetzung weiter zwischen 0 % und 5 % Fett, zwischen 3 % und 10 % Asche, zwischen 0 % und 30 % Kohlenhydrate und zwischen 0 % und 10 % Feuchtigkeit, bezogen auf das Gewicht der Zusammensetzung, umfasst.

9. Milchproteinzusammensetzung für die Verwendung nach Anspruch 8, worin die Zusammensetzung zwischen 0 % und 30 % Lactose, bezogen auf das Gewicht der Kohlenhydrate, umfasst.

10. Milchproteinzusammensetzung für die Verwendung nach einem vorstehenden Anspruch, worin die lokale Entzündung eine niedriggradige lokale Entzündung im Fettgewebe ist und vom Fettgewebe gesteuert wird.

11. Verwendung der Milchproteinzusammensetzung nach einem der Ansprüche 1 bis 10 für die Herstellung einer Nährstoffzusammensetzung oder einer pharmazeutischen Zusammensetzung für die Behandlung oder Prävention von lokaler Entzündung bei Menschen.

12. Nährstoffzusammensetzung für die Verwendung bei der Behandlung oder Prävention von chronischer lokaler Entzündung bei Menschen, die die Milchproteinzusammensetzung nach einem der Ansprüche 1 bis 10 umfasst.

13. Nährstoffzusammensetzung für die Verwendung bei der Behandlung oder Prävention von chronischer lokaler Entzündung bei Menschen nach Anspruch 12, worin die Nährstoffzusammensetzung aus der Gruppe ausgewählt ist, die ein Getränkeprodukt, ein Dessertprodukt, ein Süßwarenprodukt, ein gebackenes Produkt, ein Milchprodukt umfasst.

14. Pharmazeutische Zusammensetzung für die Verwendung bei der Behandlung oder Prävention von chronischer lokaler Entzündung bei Menschen, die die Milchproteinzusammensetzung nach einem der Ansprüche 1 bis 10 umfasst.

## Revendications

1. Composition de protéines de lait pour une utilisation dans le traitement ou la prévention d'inflammations locales chroniques chez l'homme, **caractérisée en ce que** la composition comprend au moins 12,5% d'une protéine de lait à digestion lente en poids de la composition, ladite protéine de lait à digestion lente comprenant au moins 50% de caséine en poids de la protéine sous forme micellaire.

2. Composition de protéines de lait pour une utilisation selon la revendication 1, où
la composition comprend au moins 25%, préférablement entre 25% et 95% d'une protéine de lait en poids de la composition ; et où
la protéine de lait comprend au moins 50%, préférablement entre 50% et 99%, encore plus préférablement entre 70% et 90%, de la protéine de lait à digestion lente en poids de la protéine de lait.

3. Composition de protéines de lait pour une utilisation selon l'une quelconque des revendications précédentes, où la protéine de lait à digestion lente comprend de la caséine, préférablement où la caséine se trouve sous forme micellaire et comprend un ou plusieurs parmi une caséine micellaire, du caséinate de calcium et du caséinate de magnésium.

4. Composition de protéines de lait pour une utilisation selon la revendication 3, où la caséine comprend un ou plusieurs parmi du caséinate de sodium et du caséinate de potassium.

5. Composition de protéines de lait pour une utilisation selon les revendications 3 ou 4, où la caséine comprend entre 40% et 100% d'α-caséine, entre 0% et 50% de β-caséine et entre 0% et 20% de κ-caséine, en poids de la caséine.

6. Composition de protéines de lait pour une utilisation selon l'une quelconque des revendications 3 à 5, où la caséine comprend entre 53,5% et 79,2% d'α-caséine, entre 32,1% et 45,8% de β-caséine et entre 10,7% et 16,7% de κ-caséine, en poids de la caséine.

7. Composition de protéines de lait pour une utilisation selon l'une quelconque des revendications 2 à 6, où la composition comprend en outre des protéines de lactosérum selon une quantité comprise entre 1 % et 50% en poids de la protéine de lait.

8. Composition de protéines de lait pour une utilisation selon l'une quelconque des revendications précédentes, où la composition comprend en outre entre 0% et 5% de matières grasses, entre 3% et 10% de cendres, entre 0% et 30% de glucides et entre 0% et 10% d'humidité, en poids de la composition.

9. Composition de protéines de lait pour une utilisation selon la revendication 8, où la composition comprend entre 0% et 30% de lactose en poids des glucides.

10. Composition de protéines de lait pour une utilisation selon l'une quelconque des revendications précédentes, où l'inflammation locale est une inflammation locale mal différentiée dans et dirigée par le tissu adipeux.

11. Utilisation de la composition de protéines de lait selon l'une quelconque des revendications 1 à 10, pour l'élaboration d'une composition nutritionnelle ou d'une composition pharmaceutique pour le traitement ou la prévention d'inflammations locales chez l'homme.

12. Composition nutritionnelle pour une utilisation dans le traitement ou la prévention d'inflammations locales chroniques chez l'homme, comprenant la composition de protéines de lait selon l'une quelconque des revendications 1 à 10.

13. Composition nutritionnelle pour une utilisation dans le traitement ou la prévention d'inflammations locales chroniques chez l'homme selon la revendication 12, où la composition nutritionnelle est choisie dans le groupe constitué par un produit de boisson, un produit de dessert, un produit de confiserie, un produit de panification, un produit laitier.

14. Composition pharmaceutique pour une utilisation dans le traitement ou la prévention d'inflammations locales chroniques chez l'homme, comprenant la composition de protéines de lait selon l'une quelconque des revendications 1 à 10.
